Europäisches Patentamt

European Patent Office

Office européen des brevets

(11) Veröffentlichungsnummer: **0 309 662 B1**

## (12) EUROPÄISCHE PATENTSCHRIFT

(45) Veröffentlichungstag der Patentschrift: **03.06.92**

(21) Anmeldenummer: **88111212.2**

(22) Anmeldetag: **13.07.88**

(51) Int. Cl.⁵: $A61F\ 2/36$

(54) **Oberschenkelteil einer Hüftgelenkendoprothese.**

(30) Priorität: **18.09.87 DE 8712607 U**

(43) Veröffentlichungstag der Anmeldung:
**05.04.89 Patentblatt 89/14**

(45) Bekanntmachung des Hinweises auf die
Patenterteilung:
**03.06.92 Patentblatt 92/23**

(84) Benannte Vertragsstaaten:
**AT CH DE ES FR GB IT LI NL**

(56) Entgegenhaltungen:
**EP-A- 0 065 481    EP-A- 0 243 585
DE-A- 2 851 598    DE-A- 2 933 271
DE-A- 2 941 265    DE-A- 3 006 179**

(73) Patentinhaber: **Howmedica GmbH
Professor-Küntscher-Str. 1-5
W-2314 Schönkirchen ü. Kiel(DE)**

(72) Erfinder: **Täger, Karl Heinrich, Prof. Dr. med.
Fichtenstrasse 1
W-8035 Gauting(DE)**
Erfinder: **Harder, Hans Erich
Mecklenburger Strasse 37
W-2316 Probsteierhagen(DE)**

(74) Vertreter: **Graalfs, Edo, Dipl.-Ing. et al
Patentanwälte Dipl.-Ing. H. Hauck; Dipl.-Phys.
W. Schmitz; Dipl.-Ing. E. Graalfs; Dipl.-Ing. W.
Wehnert; Dr.-Ing. W. Döring; Heidi Reichert,
Rechtsanwalt Neuer Wall 41
W-2000 Hamburg 36(DE)**

**Beschreibung**

Die Erfindung bezieht sich auf ein Oberschenkelteil einer Hüftgelenkendoprothese nach dem Oberbegriff des Patentanspruchs 1.

Beim Einsatz bekannter Endoprothesen für das Hüftgelenk besteht die grundsätzliche Alternative, den Schaft des Oberschenkelteils im Femurkanal mit oder ohne Knochenzement zu verankern. Die Verwendung von Knochenzement ermöglicht eine sehr innige und feste Verbindung zwischen dem Schaft und dem Knochen. Es besteht jedoch die Gefahr, daß durch sogenannte Mikrobewegungen eine Lockerung des Schaftes im Knochenzement eintritt, die bis zur Funktionsunfähigkeit der Endoprothese führen kann. Eine Reoperation ist dann erforderlich. Mit Knochenzement implantierte Oberschenkelteile lassen sich jedoch nur mit erheblicher Mühe operativ entfernen.

Bei zementlosen Schäften wird versucht, einen Kraftschluß zwischen Schaft und Knochen zu erzeugen. Hierzu ist erforderlich, daß der Schaft mit beträchtlicher Spannung im Knochenkanal sitzt. Ein Entfernen eines derartigen Schaftes stellt sich einfacher dar als ein mit Zement implantierter Schaft. Ein zementloser Schaft stellt jedoch für den Knochen eine höhere Belastung dar, so daß es zu Beschädigungen des Knochens beim Implantieren kommen kann. Um eine innigere Verbindung zwischen einem zementlos implantierten Schaft und dem Knochen zu erhalten, ist auch bekannt, den Schaft mit Oberflächenrauhigkeiten, Vertiefungen oder dergleichen zu versehen oder seiner Oberfläche eine vorgegebene geometrische Struktur zu erteilen. Die Knochensubstanz soll nach der Implantation in die Vertiefungen und Unebenheiten hineinwachsen und dadurch die Fixierung des Schaftes bzw. die Lastübertragung verbessern.

Aus der EP-A-0 065 481 ist eine Hüftgelenkprothese mit gelochtem Hohlschaft bekanntgeworden. Der Hohlschaft ist aus Flachmaterial gefertigt, das in die gewünschte Querschnittsform gebogen und mit Hilfe geeigneter Vorkehrungen am Prothesenhals befestigt wird. Ferner sind Mittel vorgesehen, um die aneinanderstoßenden Enden des Flachmaterials zusammenzuhalten. Hauptziel einer derartigen Prothese ist die Anpassung ihres Elastizitätsverhaltens an die Elastizität des Knochens. Ferner ermöglicht die Ausbildung von Öffnungen im Prothesenschaft das Einwachsen von Knochenmaterial sowie das Verwachsen des Knochens mit Spongiosa, die in den Hohlschaft eingefüllt wird.

Der Erfindung liegt die Aufgabe zugrunde, eine zementfrei implantierbare Hüftgelenkendoprothese zu schaffen, deren Oberschenkelteil eine größtmögliche Verbindung zwischen dem Oberschenkelteil und dem Knochen gewährleistet bei ausreichender Stabilität der Prothese. Ferner soll eine einfache Herstellung möglich sein.

Diese Aufgabe wird erfindungsgemäß durch die Merkmale des Kennzeichnungsteils des Patentanspruchs 1 gelöst.

Beim erfindungsgemäßen Oberschenkelteil ist der Schaft als Gußteil einer Chromnickelkobaltlegierung oder Titan geformt. Sie läßt sich daher auf einfache Weise aus körperverträglichem Material herstellen. Der hohle Schaft des erfindungsgemäßen Oberschenkelteils ist mit annähernd parallelen dorsalen und ventralen Seiten versehen, während die mediale und die laterale Seite im Querschnitt bogenförmig gerundet ist. Dadurch erhält der Schaft ein hohes Trägheitsmoment und damit eine ausreichende Stabilität, was für die Hohlkörperausbildung von Bedeutung ist. Herkömmliche Prothesen sind normalerweise mit einem massiven Schaft versehen. Die mit dem Schaftinneren in Verbindung stehenden Öffnungen sind ausschließlich an der dorsalen und der ventralen Seite geformt. Für die Festigkeit ist im wesentlichen die mediale und die laterale Seite verantwortlich, die auf Zug bzw. Druck beansprucht werden. Hier wäre eine Schwächung durch Löcher nachteilig. Die Anbringung der Löcher an der dorsalen oder ventralen Seite beeinträchtigt die Stabilität auch dann nur geringfügig, wenn der Öffnungsquerschnitt verhältnismäßig groß gewählt wird.

Bei dem erfindungsgemäßen Oberschenkelteil wechseln kreisrunde und längliche Öffnungen einander ab, gewährleisten mithin einen größtmöglichen Öffnungsquerschnitt zwecks wirksamen Einwachsens von Knochenmaterial bzw. Zusammenwachsens von Spongiosa im Schaftinneren und Knochen außerhalb des Schafts. Dem gleichen Zweck dient die Ausbildung einer zweiten Öffnungsreihe im proximalen Teil des Schaftes, der naturgemäß in der Lateral-Medialebene einen größeren Durchmesser im Verhältnis zum übrigen Bereich aufweist.

Der erfindungsgemäße Oberschenkelteil sichert mithin eine größtmögliche Verbindung zwischen den Knochenmaterialien innerhalb und außerhalb des Schaftes. Der Chirurg kann bei der Operation körpereigene autologe und heterologe Spongiosa einfüllen. Denkbar ist auch das Einfüllen von Hydxroxylapatit, einem Material, das für künstliche Zahnwurzeln verwendet wird. Die im Kohlkörper des Schafts und den Öffnungen befindliche Spongiosa ermöglicht ein Einheilen der Prothese im Femur durch echte knöcherne Verbindung, so daß eine sichere Verankerung erhalten wird, die auch über einen längeren Zeitraum ungefährdet ist. Die Ausbildung des Schaftes und die Anordnung der Öffnungen sichert eine ausreichende Stabilität des Schaftes.

Nach einer Ausgestaltung der Erfindung ist die Breite der Öffnungen im distalen Bereich des Schaftes größer als die Hälfte, vorzugsweise zwei Drittel der Schaftabmessung, in der lateralen/medialen Ebene. Dadurch ist ein großer Öffnungsquerschnitt geschaffen, der für die gewünschte knöcherne Verbindung der Knochenmaterialien von größtem Vorteil ist.

Um die Kerbwirkung zu verringern, sind nach einer Ausgestaltung der Erfindung die Kanten der Öffnungen gerundet.

Eine weitere Ausgestaltung der Erfindung sieht vor, daß die Öffnungen in den gegenüberliegenden Seiten einander gegenüberliegen. Diese Anordnung hat den Vorteil, daß die Öffnungen Schrauben aufnehmen können, zum Beispiel zur Anbringung einer Platte an der Außenseite des Knochens.

Die Erfindung wird nachfolgend anhand von Zeichnungen näher erläutert.

Fig. 1 zeigt schematisch die Seitenansicht eines Oberschenkelteils der Endoprothese nach der Erfindung.

Fig. 2 zeigt einen Schnitt durch die Darstellung nach Fig. 1 entlang der Linie 2-2.

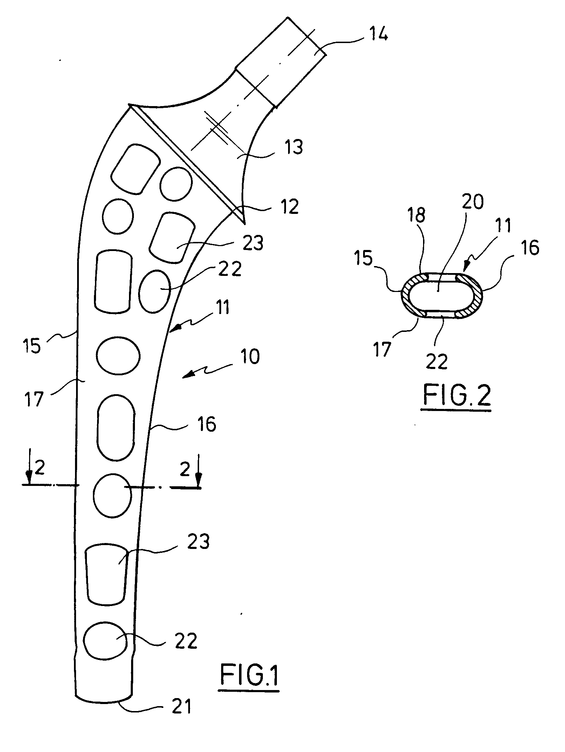

In Fig. 1 ist ein Oberschenkelteil 10 dargestellt. Er besteht aus einem relativ langen schlanken Schaft 11, einem Kragen 12 und einem sich konisch verjüngenden Hals 13, der am Ende einen Konus 14 aufweist zur Aufnahme einer Gelenkkugel, die einen entsprechenden passenden Innenkonus aufweist. Der Winkel zwischen Schaft- und Halsachse beträgt 135°.

Der Schaft 11 weist eine laterale Seite 15 und eine mediale Seite 16 auf. Es sei angenommen, daß die andere sichtbare Seite 17 des Schaftes die frontale ist. Die dorsale Seite ist mit 18 bezeichnet (Fig. 2). Man erkennt aus Fig. 2, daß die frontale und dorsale Seite 17 bzw. 18 annähernd parallel verlaufen und daß mediale Seite 16 und laterale Seite 15 bogenförmig gekrümmt sind. Von besonderer Bedeutung ist indessen, daß der Schaft 11 einen Hohlraum 20 aufweist, der sich kanalförmig durch die Länge des gesamten Schaftes erstreckt. Der Hohlraum 20 ist zum distalen Ende 21 des Schaftes 11 hin geöffnet. Die frontale Seite 17 des Schaftes 11 ist mit einer Reihe von Öffnungen versehen, die sich vom distalen bis zum proximalen Ende des Schaftes 11 erstrecken, wobei sich kreisförmige Öffnungen 22 mit länglichen Öffnungen 23 abwechseln. Die Öffnungen 22 haben einen Durchmesser, der größer ist als die Hälfte der Breite des Schaftes bzw. der frontalen Seite. Die Breite der Öffnungen 23 entspricht dem Durchmesser der Öffnungen 22. Die Länge der Öffnungen ist annähernd so groß wie der doppelte Durchmesser der Öffnungen 22. Im proximalen Bereich ist eine zweite Reihe von Öffnungen 22 bzw. 23 vorgesehen. Man erkennt, daß ein großer Flächenanteil der frontalen Seite 17 gelocht ist. Er kann größer sein als der Anteil der ungelochten Fläche. Die gegenüberliegende Rückseite 18 des Schafts 11 ist in entsprechender Weise wie in Fig. 1 gezeigt mit Löchern versehen, wobei diese vorzugsweise versetzt zu den Löchern der Seite 17 liegen.

Der gezeigte Schaft 11 ist hohl ausgeführt, da er mit körpereigener oder heterologer Spongiosa oder einer ähnlichen das Einwachsen des Schaftes fördernden Substanz gefüllt werden soll. Die Löcher oder Öffnungen 22, 23 ermöglichen damit das Zusammenwachsen der Spongiosa im Schaft 11 mit der Spongiosa im Knochenkanal bzw. der übrigen Knochensubstanz.

Im Ausführungsbeispiel wird der Schaft 11 als durchgehend hohl dargestellt und beschrieben. Es versteht sich, daß es unter Umständen auch ausreichen kann, den Schaft 11 nur teilweise hohl zu formen, um die gewünschten Wirkungen zu erhalten.

**Patentansprüche**

1. Oberschenkelteil einer Hüftgelenkendoprothese, mit einem hohlen, zum distalen Ende hin geöffneten Schaft (11), dessen mediale und laterale Seite (16, 15) im Querschnitt bogenförmig gerundet ist und dessen Wandung mehrere kreisrunde und längliche Öffnungen (22, 23) aufweist, die mit dem Schafthohlraum (20) verbunden sind, dadurch gekennzeichnet, daß der Schaft (11) ein Gußteil aus einer Chromnickelkobaltlegierung oder Titan ist, daß die dorsale Seite (18) und die ventrale Seite (17) des Schaftes (11) annähernd parallel verlaufen und ausschließlich diese dorsale und ventrale Schaftseite mit den Öffnungen (22, 23) versehen ist, wobei die kreisrunden und länglichen Öffnungen (22, 23) einander abwechseln und wobei vom distalen Schaftende (21) ausgehend eine Öffnungsreihe vorgesehen ist, neben der im proximalen Bereich eine einzige weitere Reihe von Öffnungen (22, 23) vorgesehen ist.

2. Oberschenkelteil nach Anspruch 1, dadurch gekennzeichnet, daß die Breite der Öffnungen (22, 23) im distalen Bereich des Schaftes (11) größer ist als die Hälfte, vorzugsweise zwei Drittel der Schaftabmessung in der lateralen/medialen Ebene.

3. Oberschenkelteil nach Anspruch 1 oder 2, dadurch gekennzeichnet, daß die Kanten der Öffnungen (22, 23) gerundet sind.

4. Oberschenkelteil nach einem der Ansprüche 1 bis 3, dadurch gekennzeichnet, daß die Öffnungen (22, 23) in den gegenüberliegenden Seiten (17, 18) einander gegenüberliegen.

## Claims

**1.** A femoral portion of a hip joint prosthesis, comprising a hollow shaft (11) opening towards its distal end, said shaft having a medial and lateral side (16, 15) arcuately rounded in cross-section, and having a wall including a plurality of circular and elongated openings (22, 23) which open into the hollow space (20) of the shaft, characterized in that the shaft (11) is a castings made of a chromium nickel cobalt alloy or titan, that the dorsal side (18) and the vental side (17) of the shaft (11) approximately extend parallel and that the said dorsal and ventral shaft side merely is provided with said openings (22, 23), wherein the circular and elongated openings (22, 23) alternate and wherein a row of openings is provided extending from the distal shaft end (21), alongside of which row a single further row of openings (22, 23) is provided in the proximal area.

**2.** The femoral portion of claim 1, characterized in that the width of the openings (22, 23) in the distal area of the shaft (11) is larger than the half, preferably larger than two third of the shaft dimension in the lateral/medial plane.

**3.** The femoral portion of claim 1 or 2, characterized in that the edges of the openings (22, 23) are rounded.

**4.** The femoral portion of one of claims 1 to 3, characterized in that the openings (22, 23) provided in the opposite side (17, 18) are aligned with respect to each other.

## Revendications

**1.** Partie fémorale d'une endoprothèse pour l'articulation de la hanche, comportant une tige (11) creuse, ouverte vers l'extrémité distale, dont les faces médiale et latérale (16,15) ont une section courbée en formant un arc et dont les parois présentent plusieurs ouvertures (22, 23) circulaires et allongées, qui sont reliées à la cavité intérieure (20) de la tige, caractérisée en ce que la tige (11) est une pièce de fonderie en alliage de chrome-nickel-cobalt ou en titane, en ce que la face dorsale (18) et la face ventrale (17) de la tige (11) sont dirigées sensiblement parallèlement et qu'exclusivement ces faces dorsale et ventrale de la tige sont munies d'ouvertures (22, 23), étant entendu que les ouvertures circulaires et allongées (22, 23) sont prévues en alternant les unes et les autres et qu'il est prévu une rangée d'ouvertures, partant de l'extrémité distale (21), et, à côté de cette rangée, il est prévu dans la zone proximale, une autre unique rangée d'ouvertures (22, 23).

**2.** Partie fémorale suivant la revendication 1, caractérisée en ce que la largeur des ouvertures (22, 23) dans la zone distale de la tige (11) est plus grande que la moitié, de préférence que les deux tiers, de la dimension de la tige dans le plan latéral-médial.

**3.** Partie fémorale suivant la revendication 1 ou la revendication 2, caractérisée en ce que les arêtes des ouvertures (22, 23) sont arrondies.

**4.** Partie fémorale suivant l'une quelconque des revendications 1 à 3, caractérisée en ce que les ouvertures (22, 23) se trouvent en face les unes des autres dans les faces opposées (17, 18).

FIG.1

FIG.2